(19) Europäisches Patentamt
European Patent Office
Office européen des brevets

(11) Veröffentlichungsnummer: **0 031 435**
**A1**

(12) # EUROPÄISCHE PATENTANMELDUNG

(21) Anmeldenummer: **80107076.4**

(22) Anmeldetag: **15.11.80**

(51) Int. Cl.³: **C 07 D 301/28**, C 08 G 59/06, C 09 D 3/58, C 09 J 3/16

(30) Priorität: **24.11.79 DE 2947469**

(43) Veröffentlichungstag der Anmeldung: **08.07.81 Patentblatt 81/27**

(84) Benannte Vertragsstaaten: **AT BE CH DE FR GB IT LI NL SE**

(71) Anmelder: **HOECHST AKTIENGESELLSCHAFT, Zentrale Patentabteilung Postfach 80 03 20, D-6230 Frankfurt/Main 80 (DE)**

(72) Erfinder: **Wojtech, Bernhard, Dr., Kelkheimer Strasse 67, D-6232 Bad Soden am Taunus (DE)**
Erfinder: **Kiessling, Hans-Joachim, Dr., Saselbergweg 20, D-2000 Hamburg 65 (DE)**
Erfinder: **Becker, Wilhelm, Dr., Öjendorfer Höhe 37c, D-2000 Hamburg 74 (DE)**
Erfinder: **Hermann, Kurt, Grunerstrasse 15, D-6270 Idstein/Taunus (DE)**

(54) **Verfahren zur Herstellung von Glycidyläthern ein- oder mehrwertiger Phenole, diese Glycidyläther und ihre Verwendung.**

(57) Glycidyläther ein- oder mehrwertiger Phenole von höchster Reinheit werden erhalten, wenn man die Halogenwasserstoffabspaltung aus den Halogenhydrinäthern der Phenole in wäßrigen Alkalien in Gegenwart quartärer Ammoniumverbindungen mit mindestens einem aliphatischen $C_{4-22}$-Kohlenwasserstoffrest, quartärer Phosphoniumverbindungen oder tertiärer Sulfoniumverbindungen als Katalysator durchführt oder in Gegenwart solcher Verbindungen, die im Reaktionsmedium «in situ» vor der Zugabe des Alkalis solche Oniumverbindungen aus den Halogenhydrinäthern und tertiären Aminen, tertiären Phosphinen oder Thioäthern bilden. Gegenüber den bekannten Verfahren läßt sich dadurch die Dehydrohalogenierung um ein Vielfaches beschleunigen, wodurch eine Produktionssteigerung erzielt wird. Außerdem werden durch Vermeidung von Nebenreaktionen bedeutend reinere Umsetzungsprodukte erhalten. Diese Glycidyläther finden Verwendung als niedrigviskose Gieß- und Lackharze in Form von Lacken, Klebstoffen, Formmassen usw., wobei übliche Härter eingesetzt werden können. Durch die niedrige Viskosität ist Verarbeitbarkeit erheblich besser sowie Aufnahmefähigkeit für Füllstoffe größer. Der extrem niedrige Gehalt an leicht verseifbarem Halogen bewirkt besonders günstiges Korrosionsverhalten.

EP 0 031 435 A1

0031435

HOECHST AKTIENGESELLSCHAFT HOE 79/F 319 Dr.K/jk

Verfahren zur Herstellung von Glycidyläthern ein- oder mehrwertiger Phenole, diese Glycidyläther und ihre Verwendung

Die Erfindung betrifft ein Verfahren zur Herstellung von Glycidyläthern ein- oder mehrwertiger Phenole durch Dehydrohalogenierung von Reaktionsprodukten, die aus ein- oder mehrwertigen Phenolen und Epihalogenhydrin durch Alkalien in Gegenwart spezifischer Katalysatoren erhalten worden sind, diese Glycidyläther und ihre Verwendung.

Es ist bekannt, ein- oder mehrwertige Phenole mit überschüssigem Epichlorhydrin zu Chlorhydrinäthern umzusetzen und in Gegenwart alkalisch reagierender Substanzen durch Chlorwasserstoffabspaltung in die entsprechenden Glycidyläther umzuwandeln. Dabei ist es einmal möglich, den Glycidyläther direkt, d. h. ohne Abtrennung des überschüssigen Epichlorhydrins aus dem Zwischenprodukt, dem Chlorhydrinäther, zu bilden (Einstufenverfahren). Das Verfahren in zwei Stufen sieht dagegen erst die Trennung des Chlorhydrinäthers vom überschüssigen Epichlorhydrin vor, worauf die Dehydrochlorierung mit Alkalilauge zum Glycidyläther erfolgt.

Beim einstufigen Verfahren kann die Dehydrochlorierung durch Natronlauge ohne vorherige Isolierung des Chlorhydrinäthers in Gegenwart von Wasser durchgeführt werden, welches das sich bildende Kochsalz auflöst und mit diesem nach Beendigung der Reaktion als Salzphase abgetrennt wird. Bei dem Verfahren nach der CH-PS 411 362 wird es jedoch als zweckmäßig erachtet, in Gegenwart von 25 bis 200 Gew.% n-Butanol zu arbeiten, um Epichlorhydrinverluste zu vermeiden. Dadurch wird jedoch die Ausbeute vermindert und der Destillationsaufwand erhöht, was das Verfahren unwirtschaftlich macht.

Es ist weiterhin bekannt, die Dehydrochlorierung des katalytisch vorgebildeten Chlorhydrinäthers durch eine Alkalibehandlung mit einer wäßrigen Alkalilösung durchzuführen, die 0,5 bis 0,98 Äquivalente Alkaliverbindung pro phenolische Hydroxylgruppe enthält und deren Konzentration nicht größer ist als die Sättigungskonzentration des bei der Alkalibehandlung entstehenden Salzes bei der jeweiligen Temperatur des Reaktionsgemisches. Das Verätherungsgemisch wird nach diesem Verfahren mit der wäßrigen Alkalilösung bei Temperaturen von mindestens 50°C behandelt, die wäßrige Phase abgetrennt, das Epichlorhydrin von der organischen Phase abdestilliert und der Rückstand mit wäßriger Alkalilösung nachbehandelt (vgl. DE-OS 24 07 092).

Bei anderen einstufigen Verfahren wird während der Alkalizugabe kontinuierlich das z. B. durch Natronlauge zugeführte und das durch die Reaktion gebildete Wasser durch azeotrope Destillation mit oder ohne Rückführung des als Schleppmittel benutzten überschüssigen Epichlorhydrins entfernt.

Beispielsweise wird die Dehydrochlorierung gemäß DE-AS 1 016 273 so durchgeführt, daß man eine wäßrige Lösung von mindestens 15 Gew.% Alkalihydroxyd zu einer Lösung eines mehrwertigen Phenols in wenigstens 3 Mol Epichlorhydrin je phenolischem Hydroxyläquivalent des Phenols gibt, das Wasser und Epichlorhydrin aus der Reaktionsmischung abdestilliert, die Destillationsprodukte voneinander trennt und das Epichlorhydrin der Reaktionsmischung wieder zuführt. Die Zuführungsgeschwindigkeit der Alkalihydroxidlösung und die Destillationsgeschwindigkeit werden derart geregelt, daß die Reaktionsmischung etwa 0,3 bis 2 Gew.% Wasser enthält.

In ähnlicher Weise, jedoch unter vorheriger Bildung des Chlorhydrinäthers, kann nach der DE-OS 20 28 136 gearbeitet werden, wenn man in erster Stufe überschüssiges Epichlorhydrin mit einem Polyphenol in Gegenwart eines Katalysators, z. B. eines quartären Ammoniumsalzes,

mindestens zu 5 %, bezogen auf die phenolischen OH-Gruppen, zum Chlorhydrinäther umsetzt. Im zweiten Schritt wird eine wäßrige Natriumhydroxidlösung hinzugegeben, die 0,80 bis 0,99 Äquivalente Natriumhydroxid pro phenolische OH-Gruppe enthält, wobei Wasser azeotrop abdestilliert wird. Der Glycidyläther wird dann einer Nachdehalogenierung unterworfen. Die angegebenen Gehalte an leicht verseifbarem Chlor für die erhaltenen Verfahrensprodukte liegen zwischen 0,20 und 0,75 Gew.%.

Bei diesen einstufigen Verfahren entstehen immer Nebenprodukte durch die Einwirkung des Alkalis auf das Epichlorhydrin, die die Glycidyläther verunreinigen und zu Epichlorhydrinverlusten führen.
Beim zweistufigen Verfahren versucht man die Schwierigkeiten, die sich aus den Nebenreaktionen des Epichlorhydrins im alkalischen Medium ergeben, in der Weise zu umgehen, daß man erst das ein- oder mehrwertige Phenol mit dem Epichlorhydrin in praktisch wasserfreiem Medium katalytisch umsetzt, dann das überschüssige Epichlorhydrin abdestilliert und anschließend in einer zweiten Stufe nach Zugabe eines Lösungsmittels den Chlorhydrinäther mit Alkalilauge in den Glycidyläther umwandelt. Es werden z.B. nach der US-PS 3,336,342 mehrwertige Phenole mit Epihalogenhydrinen in Gegenwart von Sulfoniumsalzen oder schwefelhaltigen Verbindungen, die mit Epihalogenhydrin zu Sulfoniumsalzen reagieren können, zu den entsprechenden Halogenhydrinen umgesetzt, aus denen nach Abtrennung des überschüssigen Epihalogenhydrins Halogenwasserstoff abgespalten wird, um zu den gewünschten Epoxidverbindungen zu gelangen. Dieses Verfahren benötigt zur Bildung der Chlorhydrinäther mindestens 40 Stunden. Das als Überschuß abdestillierte Epihalogenhydrin enthält teilweise Dihalogenhydrin und muß vor seinem wünschenswerten erneuten Einsatz gesondert aufgearbeitet werden. Das Verfahren ist sehr zeitraubend, umständlich und unwirtschaftlich.

Aus der US-PS 2,943,096 ist auch bekannt, mehrwertige

Phenole und Epichlorhydrin in Gegenwart von Kondensationskatalysatoren wie Tetramethylammoniumchlorid oder Benzyltrimethylammoniumchlorid in weitgehend wasserfreiem Medium in die entsprechenden Chlorhydrinäther umzuwandeln. Für dieses Verfahren sind 26 Stunden notwendig. Die Aufarbeitung des Reaktionsansatzes gestaltet sich sehr aufwendig. Das überschüssige, abgetrennte Epichlorhydrin muß wegen seines Gehaltes an Dichlorhydrin durch Destillation und mit Hilfe von Natronlauge zu einem für die Wiederverwendung geeigneten reineren Epichlorhydrin aufgearbeitet werden. Der isolierte, in einem Lösungsmittelgemisch gelöste Chlorhydrinäther wird durch Umsetzung mit wäßriger Natronlauge in den Glycidyläther umgewandelt. Auch hier nehmen die einzelnen Verfahrensschritte große Zeiträume in Anspruch, so daß nicht von einem sehr wirtschaftlichen Verfahren gesprochen werden kann.

In Abwandlung eines der vorstehend erwähnten Einstufenverfahren wird gemäß DE-AS 24 07 092 vom Verätherungsgemisch das gesamte Epihalogenhydrin oder ein Teil davon abdestilliert und dann der Rückstand mit wäßriger Alkalilösung behandelt. Nach Trennung der Phasen, wobei der wäßrige Anteil verworfen wird, wird von der organischen Phase das restliche und das während der Alkalibehandlung rückgebildete Epihalogenhydrin abdestilliert und der Rückstand mit wäßriger Alkalilösung nachbehandelt. Weil diese Behandlung mit der wäßrigen Alkalilösung in mehreren Stufen durchgeführt werden muß, wird das Verfahren durch die lange Kesselbelegungszeit unwirtschaftlich.

Bei allen diesen geschilderten Verfahren erhält man nach der Dehydrohalogenierung keinen reinen gebrauchsfertigen Glycidyläther der ein- oder mehrwertigen Phenole, sondern nur Rohprodukte mit einem mehr oder weniger großen Gehalt an Halogenhydrinäthern. Die Rohprodukte müssen deshalb, im allgemeinen gelöst in einem organischen Lösungsmittel, einer sogenannten Nachdehalogenierung mit

überschüssiger verdünnter wäßriger Natronlauge unterworfen werden. Dies bedeutet einen weiteren Arbeitsgang, es wird zusätzlich Abwasser erzeugt und außerdem wird ein Teil der Glycidylgruppen durch die Einwirkung des Alkalis hydrolysiert, wodurch eine Qualitätseinbuße und Verminderung der Reaktivität des Glycidyläthers im Hinblick auf seine Reaktivität hervorgerufen wird.

Die Dehydrochlorierung von Chlorhydrinäthern ein- oder mehrwertiger Phenole kann direkt in dem als Lösungsmittel dienenden überschüssigen Epichlorhydrin mit wäßrigen Alkalilösungen erfolgen. Dabei wird gleichzeitig das durch Umepoxydierung der Chlorhydrinäther mit Epichlorhydrin entstandene Dichlorhydrin wieder in Epichlorhydrin umgewandelt.

Das überschüssige Epichlorhydrin kann aber auch nach der Umsetzung der ein- oder mehrwertigen Phenole mit Epichlorhydrin aus dem Reaktionsgemisch abdestilliert werden. In diesem Fall muß das im Destillat enthaltene, aus der Umepoxydierungsreaktion stammende Dichlorhydrin vor der Wiederverwendung des Destillats durch Behandlung mit Alkalien in Epichlorhydrin umgewandelt werden. Die Reaktionsprodukte, die Chlorhydrinäther der ein- oder mehrwertigen Phenole, sind mehr oder weniger hochviskos und werden vor der HCl-Abspaltung durch wäßrige Alkalien in einem inerten, weitgehend wasserunlöslichen, organischen Lösungsmittel wie Methylisobutylketon, Benzol, Toluol und Xylol, gelöst. Das Lösungsmittel wird nach der Phasentrennung vom gebildeten Gycidyläther abdestilliert und in den Prozeß zurückgeführt. Es ist zweckmäßig, die Konzentration der eingesetzten Alkalilauge nicht über 20 % zu steigern, um Salzausfällungen zu vermeiden. Die beiden Reaktionspartner, die Chlorhydrinäther der ein- oder mehrwertigen Phenole, die in dem Lösungsmittel gelöst sind, und die wäßrigen Alkalien, die in den beiden nicht untereinander mischbaren Phasen enthalten sind, kommen nur an der Grenzfläche miteinander in Kontakt. Der Reaktionsablauf wird durch den Phasenübergang erheblich erschwert. Das macht sich besonders bei niedrigen Konzentrationen, also am Ende der Reaktion, durch stetige Abnahme der Reaktionsgeschwindigkeit störend bemerkbar. Eine Erhöhung der Phasendurchlässigkeit und eine dadurch bedingte Reaktionsbeschleunigung ist gemäß DE-AS 1 103 580 durch Zugabe einer wasserlöslichen organischen Komponente wie Äthanol, Aceton, Dioxan u. a. zum wasserunlöslichen Lösungsmittel zu erreichen. Nachteilig ist, daß durch Aufnahme eines Teils der wasserlöslichen Komponenten in die wäßrige Phase größere Lösungsmittelverluste entstehen, die zusätzliche Maßnahmen zur Rückgewinnung notwendig machen.

Es wurde nun gefunden, daß man Glycidyläther ein- oder mehrwertiger Phenole von höchster Reinheit gewinnen kann, wenn man die Halogenwasserstoffabspaltung aus den Halogenhydrinäthern der ein- oder mehrwertigen Phenole in wäßrigen Alkalien gemäß der vorliegenden Erfindung in Gegenwart von Oniumverbindungen, bevorzugt solchen mit grenzflächenaktiven Eigenschaften, z. B. kationenaktiven quartären Ammoniumverbindungen, als Katalysatoren der Halogenwasserstoffabspaltung vornimmt. Gegenüber den bekannten Verfahren läßt sich dadurch die Dehydrohalogenierung um ein Vielfaches beschleunigen, wodurch die Kesselbelegungszeit erheblich vermindert wird und durch Vermeidung von Nebenreaktionen, wie Hydrolyse schon gebildeter Glycidylgruppen, bedeutend reinere Umsetzungsprodukte erhalten werden.

Gegenstand der Erfindung ist ein Verfahren zur Herstellung von Glycidyläthern ein- oder mehrwertiger Phenole durch Umsetzung der Phenole mit Epihalogenhydrinen und anschließende Dehydrohalogenierung des Halogenhydrinäthers mit wäßrigem Alkali, bei dem die Dehydrohalogenierung in Gegenwart

a) eines Katalysators auf Basis von Oniumverbindungen, deren Liganden aus Kohlenwasserstoffresten bestehen, aus der Gruppe

1. quartäre Ammoniumverbindungen mit mindestens einem aliphatischen $C_{4}$-$_{22}$-Kohlenwasserstoffrest,
2. quartäre Phosphoniumverbindungen oder
3. tertiäre Sulfoniumverbindungen oder

b) solcher Verbindungen, die im Reaktionsmedium "in situ" vor der Zugabe des Alkalis eine solche Oniumverbindung aus den Halogenhydrinäthern und entsprechenden tertiären Aminen, tertiären Phosphinen oder Thioäthern bilden,

durchgeführt wird.

Gegenstand der Erfindung sind auch die nach dem Verfahren hergestellten Glycidyläther sowie deren Verwendung als Lack- und Gießharze.

Die Halogenhydrinäther werden durch katalytische Kondensation aus mindestens einem Phenol und Epihalogenhydrin auf übliche Weise hergestellt und sind entweder in überschüssigem Epihalogenhydrin oder, nach Abdampfen des überschüssigen Epihalogenhydrins, in einem inerten organischen Lösungsmittel gelöst.

Als ein- oder mehrwertige Phenole für die Herstellung der Halogenhydrinäther können z.B. verwendet werden: Phenol, o-, m- und p-Phenylphenol, die isomeren Amylphenole, Octylphenole und Kresole, 1,2,3-, 1,2,4-, 1,2,5-, 1,3,4- und 1,3,5-Xylenol, p-tertiär-Butylphenol, o-, m- und p-Nonylphenole, Brenzkatechin, Resorcin, Hydrochinon, 1,4- und andere Dihydroxynaphthaline, 4,4'-, 2,2'- und andere Dihydroxydiphenyle, 2,2'-, 2,4'- und 4,4'-Dihydroxydiphenylmethan, einzeln oder im Gemisch (auch als Bisphenol F bezeichnet), 4,4'-Dihydroxydibenzyl, 4,4'-Dihydroxydiphenylsulfon, ferner substituierte Dihydroxydiphenylmethane, wie sie durch saure Kondensation von Phenolen mit Aldehyden oder Ketonen entstehen, insbesondere das aus Phenol und Aceton herstellbare 4,4'-Dihydroxydiphenyl-2,2-propan, ferner Dihydroxydiphenylcyclohexan.

Als weitere Beispiele seien angeführt:

4,4'-Dihydroxy-3,3',5,5'-tetramethyl-diphenyl-methan und -2,2-propan,

4,4'-Dihydroxy-3,3',5,5'-tetra-p-tert.-butyldiphenyl-methan, -2,2-propan und -cyclohexan,

4,4'-Dihydroxy-3,3'-dimethyl-5,5'-di-p-tert.-butyldiphenyl-methan, -2,2-propan und -cyclohexan,

4,4'-Dihydroxy-3,3',5,5'-tetraamyl-diphenylcyclohexan,

Die als Ausgangsstoffe verwendeten mehrwertigen Phenole können neben den phenolischen Hydroxylgruppen auch noch andere Substituenten oder funktionelle Gruppen im Molekül enthalten, z. B. Kohlenwasserstoffreste, Äthergruppen, Estergruppen, Halogenatome, Hydroxylgruppen und andere, sofern dadurch die Reaktion nicht gestört wird. Danach kommen in Frage z. B. Tetrabrombisphenol, Tetrachlorbisphenol, Chlorhydrochinone, Methylresorcin und Phloroglucin.

Auch sind mehrwertige Phenole, z. B. Novolak-Harze, die durch säurekatalysierte Kondensation von Phenol, p-Kresol oder anderen substituierten Phenolen mit Aldehyden, wie Formaldehyd, Acetaldehyd, Crotonaldehyd, i-Butyraldehyd, i-Nonylaldehyd usw., erhalten werden, Kondensate von Phenolen mit Cardanol, Kondensate von Phenolen mit aliphatischen Diolen und Kondensate von Phenolen mit ungesättigten fetten Ölen, geeignet.

Die vorstehende Aufstellung der als Ausgangsstoffe geeigneten Verbindungen ist z. B. in dem Buch "Epoxydverbindungen und Epoxydharze" von A. M. Paquin, Springer-Verlag, 1958, Seiten 256 - 307, enthalten. Bevorzugt werden Phenol, p-tertiär-Butylphenol, 4,4'-Dihydroxydiphenyl-2,2-propan oder -methan, Tetrabrombisphenol und Phenol-Novolake eingesetzt.

In einer anderen Ausführungsform wird eine Mischung aus 0,60 bis 0,99 Molen 4,4'-Dihydroxydiphenyl-2,2-propan und 0,40 bis 0,01 Molen eines Diphenols aus der Gruppe der

 

oben angeführten Verbindungen, insbesondere Hydrochinon, Resorcin, Bisphenol F, die vorgenannten Novolak-Harze, zur Herstellung der Diglycidyläther mit niedriger Viskosität (6000 - 16000 m Pa's/25°C) verwendet, um bei längerer Lagerung in kühlen Räumen eine Kristallisation dieser Produkte zu verhindern.

Als Epihalogenhydrine kommen z.B. Epibromhydrin oder 1,4-Dichlor-2,3-epoxybutan, vorzugsweise aber Epichlorhydrin in Frage. Das Epihalogenhydrin wirkt nicht nur als Reaktionskomponente, sondern auch als Lösungsmittel für das ein- oder mehrwertige Phenol und den gebildeten Äther.

Die Lösung des Halogenhydrinäthers wird mit verdünnter Alkalilauge sowie dem Onium-Katalysator versetzt und der Halogenhydrinäther in diesem Zweiphasensystem unter kräftigem Rühren, zweckmäßig bei erhöhter Temperatur durch Halogen-Abspaltung zu Glycidyläthern umgesetzt.

Die quartären Ammoniumverbindungen zählen zu den am leichtesten zugänglichen und daher gebräuchlichsten kationenaktiven Oniumverbindungen. Sie sind grenzflächenaktive (oberflächenaktive) Stoffe, bei denen sich der die Grenzflächenaktivität bedingende, höhermolekulare hydrophobe Rest bei einer Dissoziation in wäßriger Lösung im Kation befindet. Alle kationenaktiven Verbindungen enthalten eine basische Oniumgruppe, z. B. basischen Stickstoff im Ammoniumion mit einem oder mehreren höhermolekularen hydrophoben Resten. Sie reichern sich beim Auflösen in Wasser bevorzugt an der Oberfläche bzw. in Gegenwart einer lipophilen Phase an der Phasengrenzfläche an.

Die Katalysatoraktivität beim erfindungsgemäßen Verfahren steigt beispielsweise mit wachsender Kettenlänge der Liganden am Ammoniumstickstoff. So sind Tetramethylammoniumchlorid wie auch Trimethylbenzylammoniumchlorid, die bei der Umsetzung von Phenolen und Epichlorhydrin bevorzugten Kondensationskatalysatoren, wesentlich weniger

aktiv als beispielsweise Tetrabutylammoniumchlorid. Dieses ist wiederum nicht so wirksam wie kationenaktive Verbindungen mit längerkettigen Alkylresten, z. B. Distearyldimethylammoniumchlorid oder Cocosalkyldimethylbenzylammoniumchlorid.

Als Dehydrohalogenierungskatalysatoren geeignet Ammoniumverbindungen haben die allgemeine Formel

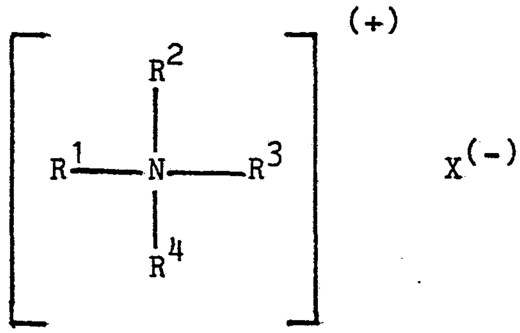

(I),

in der die Reste $R^1$, $R^2$, $R^3$ und $R^4$ gleich oder verschieden sind, mit der Maßgabe, daß höchstens drei Reste je eine Alkyl-, Cycloalkyl-, Alkenyl-, Aryl- oder Aralkylgruppe mit bis zu 18, vorzugsweise 1 bis 12 C-Atomen darstellen, die auch Hydroxyl- oder Äthergruppen enthalten können, mindestens ein Rest aber eine verzweigte oder unverzweigte Alkyl- oder Alkenylgruppe mit 4 bis 22, vorzugsweise 8 bis 22 C-Atomen ist und $X^{(-)}$ ein Monoanion, vorzugsweise ein Halogenanion wie Chlorid, Bromid, Jodid oder ein Sulfonatsalz darstellt. Besonders bevorzugt wird das Chloridion.

Geeignete Phosphonium- und tertiäre Sulfoniumverbindungen haben die Formeln

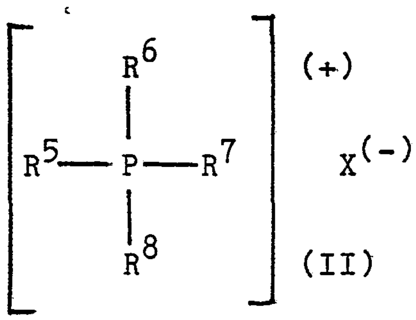

(II) und

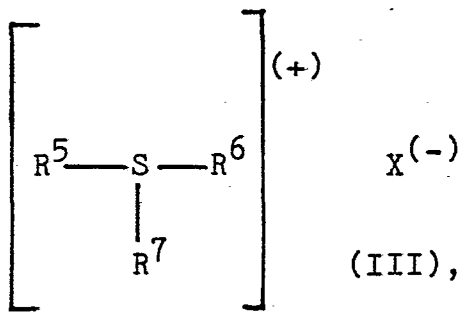

(III),

 

in denen $R^5$, $R^6$, $R^7$ und $R^8$ gleich oder verschieden sein können und Alkyl-, Cycloalkyl-, Alkenyl-, Aralkyl- oder Arylgruppen mit jeweils 1 bis 12 C-Atomen darstellen, die gegebenenfalls Hydroxyl- oder Äthergruppen enthalten und $X^{(-)}$ als Monoanion die gleiche Bedeutung wie oben hat, werden in gleicher Weise wie die quartären Ammoniumverbindungen eingesetzt.

Kationenaktive Oniumkatalysatoren lassen sich im allgemeinen durch "Peralkylierung", worunter auch die Umsetzung mit entsprechenden Arylverbindungen verstanden werden soll, von tertiären Aminen, tertiären Phosphinen und Thioäthern, insbesondere Dialkylsulfiden, erzeugen. Der höhermolekulare, hydrophobe Rest kann von den Grundsubstanzen wie auch vom Alkylierungsmittel stammen. Als "Alkylierungsmittel" können anstelle von Alkylchloriden z.B. auch Halogenalkylalkohole oder Halogenalkyläther bzw. die entsprechenden Arylverbindungen eingesetzt werden.

Es wurde gefunden, daß sich auch die Halogenhydrinäther als "Alkylierungsmittel" eignen und durch Umsetzung mit den tertiären Aminen, tertiären Phosphinen oder Thioäthern hochaktive Katalysatoren ergeben. Die Katalysatorherstellung kann deshalb "in situ" direkt im Reaktionsgefäß in vorgelegten Halogenhydrinäthern vor der Dehydrohalogenierung erfolgen. Nach der Katalysatorbildung, die zur Beschleunigung bei erhöhter Temperatur vorgenommen wird, erfolgt die Dehydrohalogenierung in Gegenwart von wäßrigen Alkalien in der üblichen Weise.

Die Katalysatormenge bei der Dehydrohalogenierung beträgt z. B., bezogen auf Halogenhydrinäther, 0,01 bis 10 Gew.%, vorzugsweise 0,1 bis 5 Gew.%.

Bevorzugte quartäre Ammoniumkatalysatoren, sind z. B. Octyltrimethyl- oder Sojaalkyltrimethyl- sowie Distearyldimethyl-ammoniumchlorid oder Verbindungen mit

einem langkettigen Rest und einer Benzylgruppe wie Cocosalkylbenzyl-dimethylammoniumchlorid.

Bevorzugte quartäre Phosphoniumverbindungen sind beispielsweise Triphenyläthylphosphoniumbromid, Tetramethyl-, Tetraphenyl- und Tetrabenzylphosphoniumchlorid.

Geeignete Sulfoniumverbindungen sind z. B. Trimethylsulfoniumjodid und Dibenzylmethylsulfoniumbromid.

Tertiäre Amine, Phosphine und Dialkylsulfide, die im Reaktionsmedium mit den Halogenhydrinäthern Oniumsalze der beanspruchten Art bilden, sind beispielsweise Tri-n-butylamin, Tri-tert.butylamin, Triisooctylamin, Octyldimethylamin, Sojalkyldimethylamin, Distearylmethylamin, Triphenylphosphin, Trimethylphosphin, Triäthylphosphin, Tripropylphosphin, Tributylphosphin, Tribenzylphosphin,Dibenzylsulfid, Benzyläthylsulfid, Benzyl-butylsulfid und Diäthylsulfid.

Die Dehydrohalogenierung erfolgt zweckmäßig mit wäßrigen Alkalien wie Natron- oder Kalilauge, vorzugsweise jedoch Natronlauge. Die Konzentration der alkalischen Lösung soll bei der Dehydrohalogenierung so gewählt werden, daß Ausfällungen von Salzniederschlägen vermieden werden, die die Phasentrennung erschweren. Vorzugsweise ist die Konzentration nicht höher als 18 Gew.%, bezogen auf Natriumhydroxyd. Im allgemeinen arbeitet man mit stöchiometrischen Mengen; zweckmäßig kann die Halogenwasserstoffabspaltung unter Verwendung eines bis zu 20%igen Überschusses über die stöchiometrisch erforderliche Menge an Base durchgeführt werden.

Maßgebend für die benötigte Alkalimenge ist der Gehalt an aktivem (verseifbarem) Halogen, z. B. Chlor im Chlorhydrinäther oder, wenn im überschüssigen Epichlorhydrin gearbeitet wird, die Anzahl der Hydroxylgruppen im phenolischen Ausgangsprodukt. Das molare Verhältnis von benötigter Alkalimenge zu hydrolysierbarem Halogen bzw. zu der Anzahl der phenolischen Hydroxylgruppen beträgt im allgemeinen 0,95 bis 1,5 : 1, vorzugsweise 1,0 bis 1,2 : 1.

Die Dehydrohalogenierung vom Halogenhydrinäther zum Diglycidyläther kann entsprechend dem bekannten Zweistufenverfahren nach dem Abdestillieren des Epichlorhydrins in einem inerten Lösungsmittel, z. B. Toluol, Xylol, höheren Ketonen wie Methylisobutylketon, höheren Alkoholen wie n- oder i-Butanol oder Gemischen dieser Lösungsmittel erfolgen.
Es ist vorteilhaft, bei erhöhter Temperatur zwischen 40° und 95°C, bevorzugt zwischen 70° und 90°C, zu arbeiten. Da es sich um eine Zweiphasenreaktion handelt, wird das Reaktionsgemisch zweckmäßig kräftig gerührt.
Wird nach dem Einstufenverfahren das überschüssige Epichlorhydrin nicht abgetrennt, ist der Zusatz eines inerten Lösungsmittels zur Verdünnung des Halogenhydrinäthers nicht erforderlich. In diesem Fall sollte die Dehydrohalogenierung bei nicht zu hoher Temperatur, vorzugsweise bei 40 bis 60°C vorgenommen werden, um Nebenreaktionen des Epihalogenhydrins im alkalischen wäßrigen Medium zu vermeiden. Das geringere Reaktionsvermögen bei tiefer Temperatur erfordert eine höhere Katalysatorkonzentration. Um eventuell auftretende Epihalogenhydrinverluste durch Nebenreaktionen möglichst klein zu halten, empfiehlt es sich weiter, die Natronlauge nur langsam in das kräftig gerührte Reaktionsgemisch einfließen zu lassen, etwa in dem Maße, wie sie umgesetzt wird. Auf diese Weise läßt sich eine zu hohe Alkalikonzentration im Reaktionsmedium vermeiden.

Das erfindungsgemäße Verfahren kann kontinuierlich und diskontinuierlich betrieben werden. Im kontinuierlichen Betrieb können die beiden Phasen (organische Phase und wäßrige Alkalilauge) im Gegenstrom und im Gleichstrom, sowohl einstufig als auch mehrstufig, geführt werden. Die Verteilung des Katalysators auf beide Phasen bringt

im Gleichstrombetrieb (Kaskadenführung) bei mehrstufiger Arbeitsweise eine bessere Katalysatorausnutzung und damit eine bessere Ausbeute als im Gegenstrombetrieb.

Nach Beendigung der Dehydrohalogenierung werden die beiden Phasen, die sich aufgrund ihres Dichteunterschiedes gesondert abscheiden, getrennt und der Glycidyläther aus der organischen Phase nach dem Abdestillieren des Lösungsmittels gewonnen. Das Lösungsmittel geht in den Prozeß zurück. Die Glycidyläther werden, vorteilhaft unter vermindertem Druck und bei Temperaturen bis 150°C, bevorzugt bis 120°C, von Lösungsmittelresten und anschließend durch Filtration von Salzresten befreit. Die Glycidyläther werden durch die Bestimmung ihrer Viskosität, ihres Epoxidäquivalentes und ihres Gehaltes an hydrolysierbarem Halogen (nach ASTM D 1226-60T) charakterisiert.

Die nach dem vorstehenden Verfahren erhaltenen Glycidyläther ein- oder mehrwertiger Phenole können für sich oder gegebenenfalls zusammen mit geeigneten Zusatzmitteln als niederviskose Gieß- und Lackharze unter Verwendung üblicher Härter wie Aminen, Polycarbonsäuren oder ihren Anhydriden als Lacke, Klebstoffe, Formmassen, usw. Verwendung finden. Infolge ihrer niedrigen Viskosität ist eine erhebliche bessere Verarbeitbarkeit und eine wesentlich größere Füllstoffaufnahmefähigkeit gegeben. Durch den extrem niedrigen Gehalt an leicht verseifbarem Halogen zeichnen sich die Glycidyläther zudem durch besonders günstiges Korrosionsverhalten aus, was besonders bei der Verarbeitung mit metallischen Gegenständen wichtig ist.

In den nachfolgenden Beispielen bedeuten T stets Teile und % stets Gewichtsprozent.

BEISPIELE

1 a) Herstellung des Bisphenol-A-dichlorhydrinäthers:

228 T 4,4'-Dihydroxydiphenyl-2,2-propan (Bisphenol A) und 920 T Epichlorhydrin wurden vermischt und mit 5,2 T einer Cholinchloridlösung (70 %ig in Wasser) je Mol Bisphenol A versetzt. Das Gemisch wurde unter Rückfluß 5 Minuten auf etwa 120°C gehalten, anschließend das überschüssige Epichlorhydrin abdestilliert und der noch verbleibende Epichlorhydrinrest unter vermindertem Druck bei 120 bis 125°C entfernt. Umsatz etwa 98 %; bezogen auf die phenolischen Hydroxylgruppen. Gehalt an hydrolysierbarem Chlor 8,3 %, Epoxidäquivalent 390 bis 410, Viskosität 80 Pa.s/ 25°C.

1 b) Dehydrochlorierung des Bisphenol-A-dichlorhydrinäthers zum Diglycidyläther:

Die 50 %ige Lösung des Dichlorhydrinäthers in Xylol wurde mit soviel 7 %iger wäßriger Natronlauge versetzt, daß das molare Verhältnis NaOH/hydrolysierbares Chlor 1,09/1,0 betrug. Der Natronlauge war zuvor 0,2 % Distearyldimethylammoniumchlorid (75 %ige Lösung in Isopropanol-Wasser 2 : 1), bezogen auf die eingesetzte, lösungsmittelfreie Dichlorhydrinäthermenge als Katalysator zugesetzt worden. Das Reaktionsgemisch wurde unter kräftigem Rühren 90 Minuten bei 80°C erwärmt und danach schnell auf Raumtemperatur abgekühlt. Die Phasen schieden sich nach Abschalten des Rührers rasch ab und wurden getrennt. Aus der organischen Xylolphase wurde das Xylol destillativ erst unter Normaldruck, dann unter vermindertem Druck bei steigender Temperatur bis 120°C entfernt. Der gebildete Glycidyläther enthielt 0,16 % hydrolysierbares Chlor, Epoxidäquivalent 186; Viskosität 9,03 Pa.s/25°C.

2 bis 6)

Beispiel 1 wurde wiederholt mit dem Unterschied, daß andere Katalysatoren bei der Dehydrochlorierung eingesetzt wurden. Tabelle 1 gibt die Mengenverhältnisse der Katalysatoren, bezogen auf lösungsmittelfreie Dichlorhydrinäthermenge, Versuchsbedingungen und Kenndaten der erhaltenen Glycidyläther an. In den Beispielen 5 und 6 wurde der Katalysator "in situ" erzeugt, d. h. tertiäres Amin bzw. Dialkylsulfat wurden zu der 50 %igen Lösung des Dichlorhydrinäthers gegeben und der Ansatz jeweils eine Stunde auf 80°C zur Quaternierung des tertiären Amins bzw. der Sulfoniumbildung des Thioäthers erhitzt, worauf die Natronlauge zugefügt wurde.

Tabelle 1

| Beispiel | 2 | 3 | 4 | 5 | 6 |
|---|---|---|---|---|---|
| Katalysatorart | Cocosalkylbenzyldimethylammoniumchlorid | Tetraphenylphosphoniumchlorid | Trimethylsulfoniumjodid | Triisooctylamin | Dibenzylsulfid |
| Katalysatormenge (%) | 1 | 1,4 | 1,4 | 1 | 1,4 |
| Reaktionsbedingungen | 20', 80°C | 30', 80°C | 20', 80°C | 20', 80°C | 20', 80°C |
| hydrolysierbares Chlor (%) | 0,2 | 0,12 | 0,11 | 0,03 | 0,05 |
| Epoxydäquivalent | 186 | 184 | 181 | 188 | 183 |
| Viskosität Pa.s (25°C) | 9,40 | 9,53 | 9,67 | 11,6 | 13,8 |

7) Zur kontinuierlichen Herstellung des Bisphenol-A-diglycidyläthers wurde eine Lösung von 50 % Dichlorhydrinäther in Xylol (Herstellung Beispiel 1 a)) mit 1,25 % Distearyldimethyl-ammoniumchlorid als 75 %ige Lösung in Isopropanol-Wasser (2 : 1) (bezogen auf den Dichlorhydrinäther) versetzt. Diese Speiselösung (= S) wurde anschließend zusammen mit 7%iger wäßriger Natronlauge in eine 2-stufige Rührkesselkaskade (Gleichstromführung) so eingeleitet, daß ein molares Verhältnis NaOH/hydrolysierbares Chlor von 1,1/1,0 bestand (Strömungsverhältnis S/Lauge = 1,5/1.0). Die Temperatur der beiden Rührkessel betrug 80°C, die Verweilzeit je Kessel 7 Minuten. Die Kennzahlen des entstandenen Diglycidyläthers lauten: 0,20 % hydrolysierbares Chlor, Epoxidäquivalent 188, Viskosität 9,35 Pa.s/25°C.

8) Eine Mischung aus 100 T 4,4'-Dihydroxydiphenyl-2,2'-propan, 406 T Epichlorhydrin und 2 T einer Lösung von 50 % Tetramethyl-ammoniumchlorid in Wasser (molares Verhältnis von 1:10 : 0,021) wurde unter Rühren 3,5 Stunden auf 100°C erhitzt. Dabei wurden 98% des Bisphenols umgesetzt (UV-Spektralanalyse). Das Reaktionsgemisch (Bisphenol-A-dichlorhydrinäther in überschüssigem Epichlorhydrin) wurde nun auf 50°C abgekühlt und die Dehydrochlorierung bei dieser Temperatur durch Zugabe von 287,5 T 14 %iger Natronlauge in Gegenwart von Distearyldimethyl-ammoniumchlorid als Katalysator vorgenommen. Das molare Verhältnis von eingebrachtem Natriumhydroxid zu vorgelegten phenolischen OH-Gruppen betrug 1,15 : 1. Zu Reaktionsbeginn wurden 40 T Lauge, das sind 14 % der Gesamtlaugenmenge, mit dem gesamten Katalysator, das sind 2,4 T einer Lösung von 75 % Distearyldimethyl-ammoniumchlorid in Isopropanol-Wasser (2 : 1) eingebracht. Die restlichen 86 % der Natronlauge (247,5 T) wurden innerhalb von 105 Minuten langsam unter kräftigem Rühren zugegeben und dann das Gemisch zur Vervollständigung der Reaktion noch 15 Minuten gerührt. Die Phasen, die sich nach Abschalten des Rührers bildeten, wurden getrennt und das Epichlorhydrin aus der organischen Phase unter vermindertem Druck bei einer bis 110°C ansteigenden Temperatur abdestilliert. Die Ausbeute an Diglycidyläther betrug 145 T; hydrolysierbares Chlor 0,15 %, Epoxidäquivalent 181, Viskosität 8,12 Pa.s/25°C.

9) Beispiel 8 wurde wiederholt mit 100 T p-tert.-Butylphenol, 333 T Epichlorhydrin und 3,1 T einer Lösung von 50 % Tetramethyl-ammoniumchlorid in Wasser (molares Verhältnis 1:10 : 0,021) und solange auf 100°C erhitzt, bis mindestens 98 % der phenolischen Hydroxylgruppen umgesetzt waren. Das Reaktionsgemisch wurde auf 50°C abgekühlt und die Dehydrochlorierung mit 218,5 g 14 %iger Natronlauge in Gegenwart von 2,4 T Distearyldimethyl-ammoniumchlorid (75 %ig in Isopropanol-Wasser, 2:1) durchgeführt. Ausbeute 134 T des p-tert.-Butylglycidyläthers; hydrolysierbares Chlor 0,12 %, Epoxidäquivalent 212, Viskosität von 17 mPa.s /25°C.

10) Beispiel 8 wurde wiederholt mit 100 T Bisphenol F (Isomerengemisch), 555 T Epichlorhydrin und 2,3 T einer Lösung von 50 % Tetramethyl-ammoniumchlorid in Wasser (molares Verhältnis: 1:12 : 0,021) und solange auf 100°C erhitzt, bis mindestens 98 % der phenolischen Hydroxylgruppen umgesetzt waren. Das Reaktionsgemisch wurde auf 50°C abgekühlt und die Dehydrochlorierung mit 329 T 14 %iger Natronlauge in Gegenwart von 2,4 T Distearyldimethyl-ammoniumchlorid (75%ig in Isopropanol-Wasser, 2:1) durchgeführt. Ausbeute 149 T des Glycidyläthers des Bisphenol F (Isomerengemisch); hydrolisierbares Chlor 0,17 %, Epoxidäquivalent 172, Viskosität 2120 mPa.s /25°C.

11) Beispiel 8 wurde wiederholt mit einer Mischung aus 59,5 T Tetrabrombisphenol, 40,5 T Bisphenol A, 313 T Epichlorhydrin und 1,3 T einer Lösung von 50 %

Tetramethyl-ammoniumchlorid in Wasser (molares Verhältnis: 1:12 : 0,021) und solange auf 100°C erhitzt, bis mindestens 97 % der phenolischen Hydroxylgruppen umgesetzt waren. Das Reaktionsgemisch wurde auf 50°C abgekühlt und die Dehydrochlorierung mit 188,6 T 14 %iger Natronlauge in Gegenwart von 2,4 T Distearyldimethylammoniumchlorid (75 %ig in Isopropanol-Wasser, 2:1) durchgeführt.
Ausbeute 129 T des zähflüssigen Mischglycidyläthers des Tetrabrombisphenols und Bisphenol A; hydrolysierbares Chlor 0,13 %, Epoxidäquivalent 249, Viskosität 750 Pa.s /25°C.

12) 900 T Phenol wurden mit 9 T kristallisierter Oxalsäure, gelöst in 18 T Wasser, auf 100°C erwärmt. Bei 100°C wurden am Rückfluß 369 T 37 %ige Formaldehydlösung zugesetzt und auf 120°C erwärmt, wobei unter Verwendung eines Abscheiders hauptsächlich ein wäßriges Destillat entfernt wird. Unter vermindertem Druck bei 20 mm Hg wurde bis auf 150°C erwärmt, wobei überschüssiges Phenol weitgehend entfernt wurde. Durch eine Wasserdampfdestillation unter vermindertem Druck bei 140 - 150°C wurde anschließend der Gehalt an freiem Phenol auf 0,5 % herabgesetzt.

Entsprechend den Angaben des Beispiels 8 wurden 100 T des beschriebenen Novolaks, 555 T Epichlorhydrin und 4,6 T einer Lösung von 50 % Tetramethyl-ammoniumchlorid in Wasser (molares Verhältnis: 1:12 : 0,042) solange auf 118 bis 120°C am Rückfluß erhitzt, bis mindestens 96 % der phenolischen Hydroxylgruppen umgesetzt waren. Das Reaktionsgemisch wurde auf 50°C abgekühlt und die Dehydrochlorierung mit 324 g 14 %iger Natronlauge in Gegenwart von 2,4 T Distearylmethylammoniumchlorid (75%ig in Isopropanol-Wasser, 2:1) durchgeführt. Ausbeute 145 T des Novolak-Glycidyläthers; hydrolysierbares Chlor 0,14 %, Epoxidäquivalent 173, Viskosität 23.600 mPa.s /52°C.

Vergleichsuntersuchungen zum Nachweis des erzielten technischen Fortschritts.

Die Reaktionsgeschwindigkeit der Dehydrochlorierung ohne und mit Katalysator wurde anhand des im Diglycidyläther verbliebenen hydrolysierbaren Chloranteils verglichen.

Zweistufenverfahren

Beispiel 1 wurde ohne und mit verschiedenen Katalysatoren wiederholt, wobei die Reaktionszeit während der Dehydrochlorierung im allgemeinen 20 Minuten betrug. Siehe Tabelle 2.

Tabelle 2

| Beispiel | Katalysator Art | Menge (%) | Reaktionszeit (Min.) | hydrolysierbares Chlor (%) |
|---|---|---|---|---|
| 13 | Tetrabutylammoniumchlorid | 0,20 | 20 | 0,45 |
| 14 | Distearyldimethylammoniumchlorid (75 %ig in Isopropanol-Wasser, 2:1) | 0,20 | 20 | 0,20 |
| 15 | - " - | 2,0 | 5 | 0,20 |
| Vergl. 1a | ohne Katalysator | - | 20 | 4,1 |
| Vergl. 1b | - " - | - | 500 | 1,2 |
| Vergl. 2 | Tetraäthylammoniumchlorid | 0,20 | 20 | 3,6 |
| Vergl. 3 | Benzyltrimethyl-ammoniumchlorid | 0,20 | 20 | 3,4 |

Während bei der Dehydrochlorierung ohne Katalysator auch nach 500 Minuten Reaktionszeit noch ein hoher Gehalt an hydrolysierbarem Chlor vorhanden war, ist es mit Hilfe des erfindungsgenmäßen Verfahrens mit bestimmten Katalysatoren möglich, innerhalb kurzer Zeit weitgehend chlorfreie Produkte zu erhalten. Die Überlegenheit der erfindungsgemäß zugesetzten Katalysatoren wird besonders deutlich in einer erheblichen Verkürzung der Reaktionszeit, wenn höhere Katalysatormengen eingesetzt werden. Kondensationskatalysatoren, die bei der Herstellung der Chlorhydrinäther eingesetzt werden, zeigen bei der Dehydrochlorierung nur eine geringfügige Wirksamkeit.

Einstufenverfahren

Beispiel 8 wurde ohne Katalysator wiederholt. Tabelle 3 gibt die erhaltenen Werte im Vergleich zu Beispiel 8 an.

Tabelle 3

| Beispiel | Katalysator | | Reaktionszeit (Min.) | | hydrolysierbares Chlor |
|---|---|---|---|---|---|
| | Art | Menge (%) | Laugezugabe | Gesamt | |
| 8 | Distearyldimethylammoniumchlorid (75 %ig in Isopropanol-Wasser, 2:1) | 2,40 | 105 | 120 | 0,15 |
| Vergl. 4 | ohne Katalysator | - | 105 | 120 | 2,1 |

Aus den Versuchen der Tabelle 3 geht hervor, daß durch die Anwesenheit eines Dehydrohalogenierungskatalysators bei dem Einstufenverfahren Glycidyläther mit einem sehr niedrigen Gehalt an hydrolysierbarem Chlor erhalten werden.

PATENTANSPRÜCHE

1. Verfahren zur Herstellung von Glycidyläthern ein- oder mehrwertiger Phenole durch Umsetzung der Phenole mit Epihalogenhydrin und anschließende Dehydrohalogenierung des Halogenhydrinäthers mit wäßrigem Alkali, dadurch gekennzeichnet, daß die Dehydrohalogenierung in Gegenwart

a) eines Katalysators auf Basis von Oniumverbindungen, deren Liganden aus Kohlenwasserstoffresten bestehen, aus der Gruppe
   1. quartäre Ammoniumverbindungen mit mindestens einem aliphatischen $C_4$-$C_{22}$-Kohlenwasserstoffrest,
   2. quartäre Phosphoniumverbindungen oder
   3. tertiäre Sulfoniumverbindungen oder
b) solcher Verbindungen, die im Reaktionsmedium "in situ" vor der Zugabe des Alkalis eine solche Oniumverbindung aus den Halogenhydrinäthern und entsprechenden tertiären Aminen, tertiären Phosphinen oder Thioäthern bilden,

durchgeführt wird.

2. Verfahren nach Anspruch 1, dadurch gekennzeichnet, daß die quartäre Ammoniumverbindungen die allgemeine Formel

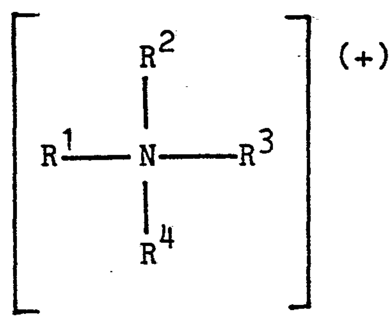 $X^{(-)}$ (I),

haben, in der die Reste $R^1$, $R^2$, $R^3$ und $R^4$ gleich oder verschieden sind, mit der Maßgabe, daß höchstens drei Reste je eine unsubstituierte, mit Hydroxylgruppen substituierte oder Äthergruppen enthaltende Alkyl-, Cycloalkyl-, Alkenyl-, Aryl- oder Aralkylgruppe mit bis zu 18, vorzugsweise 1 bis 12, C-Atomen darstellen, mindestens ein Rest aber eine Alkyl- oder Alkenylgruppe mit 4 bis 22, vorzugsweise 8 bis 22, C-Atomen ist und $X^{(-)}$ ein Monoanion, vorzugsweise das

Chloridion, darstellt.

3. Verfahren nach Anspruch 1, dadurch gekennzeichnet, daß die quartären Phosphoniumverbindungen die allgemeine Formel

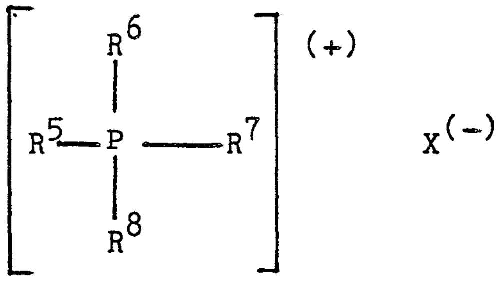

(II),

haben, in der $R^5$, $R^6$, $R^7$ und $R^8$ gleich oder verschieden sind und Alkyl-, Cycloalkyl-, Alkenyl, Aralkyl- oder Arylgruppen mit jeweils 1 - 12 C-Atomen darstellen, die gegebenenfalls Hydroxyl- oder Äthergruppen enthalten und $X^{(-)}$ ein Monoanion, vorzugsweise das Chloridion, ist.

4. Verfahren nach Anspruch 1, dadurch gekennzeichnet, daß die tertiären Sulfoniumverbindungen die allgemeine Formel

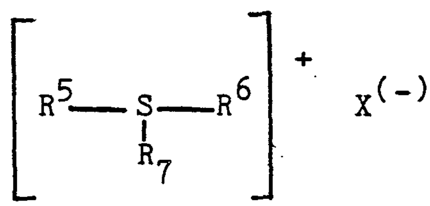

(III)

haben, in der $R^5$, $R^6$, und $R^7$ gleich oder verschieden sind und Alkyl-, Cycloalkyl-, Alkenyl, Aralkyl- oder Arylgruppen mit 1 - 12 C-Atomen darstellen, die gegebenenfalls Hydroxyl- oder Äthergruppen enthalten und $X^{(-)}$ ein Monoanion, vorzugsweise das Chloridion, ist.

5. Verfahren nach einem oder mehreren der Ansprüche 1 bis 4, dadurch gekennzeichnet, daß der Dehydrohalogenierungskatalysator in einer Menge von 0,01 bis 10, vorzugsweise 0,1 bis 5, Gew.%, bezogen auf Halogenhydrinäther, eingesetzt wird.

6. Verfahren nach einem oder mehreren der Ansprüche 1 bis 7, dadurch gekennzeichnet, daß eine Alkalimenge, vorzugsweise Natronlauge zugegeben wird, deren molares Verhältnis zu der Menge an hydrolysierbarem Halogen oder der Anzahl der Hydroxylgruppen im Ausgangsphenol 0,95 bis 1,5 : 1, vorzugsweise 1,0 bis 1,2 : 1, beträgt.

7. Verfahren nach einem oder mehreren der Ansprüche 1 bis 6, dadurch gekennzeichnet, daß die Temperatur bei der Dehydrohalogenierungsreaktion im Zweistufenverfahren 40 bis 95°C, vorzugsweise 70 bis 90°C, beträgt.

8. Verfahren nach einem oder mehreren der Ansprüche 1 bis 6, dadurch gekennzeichnet, daß die Dehydrohalogenierung beim Einstufenverfahren bei 40 bis 60°C erfolgt.

9. Glycidyläther, hergestellt nach dem Verfahren gemäß einem oder mehreren der Ansprüche 1 bis 8.

10. Verwendung der Glycidyläther nach Anspruch 9 zusammen mit geeigneten Zusatzmitteln und Härtern als Gießharze, Lacke, Klebstoffe oder Formmassen.

PATENTANSPRÜCHE FÜR ÖSTERREICH:

1. Verfahren zur Herstellung von Glycidyläthern ein- oder mehrwertiger Phenole durch Umsetzung der Phenole mit Epihalogenhydrin und anschließende Dehydrohalogenierung des Halogenhydrinäthers mit wäßrigem Alkali, dadurch gekennzeichnet, daß die Dehydrohalogenierung in Gegenwart

a) eines Katalysators auf Basis von Oniumverbindungen, deren Liganden aus Kohlenwasserstoffresten bestehen, aus der Gruppe
   1. quartäre Ammoniumverbindungen mit mindestens einem aliphatischen $C_4$-$C_{22}$-Kohlenwasserstoffrest,
   2. quartäre Phosphoniumverbindungen oder
   3. tertiäre Sulfoniumverbindungen oder

b) solcher Verbindungen, die im Reaktionsmedium "in situ" vor der Zugabe des Alkalis eine solche Oniumverbindung aus den Halogenhydrinäthern und entsprechenden tertiären Aminen, tertiären Phosphinen oder Thioäthern bilden,

durchgeführt wird.

2. Verfahren nach Anspruch 1, dadurch gekennzeichnet, daß die quartäre Ammoniumverbindungen die allgemeine Formel

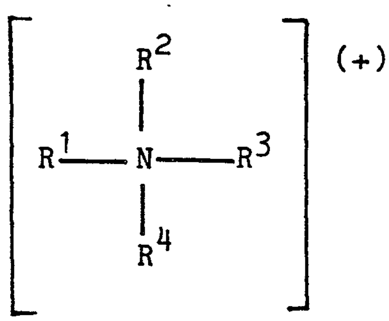 $X^{(-)}$ (I),

haben, in der die Reste $R^1$, $R^2$, $R^3$ und $R^4$ gleich oder verschieden sind, mit der Maßgabe, daß höchstens drei Reste je eine unsubstituierte, mit Hydroxylgruppen substituierte oder Äthergruppen enthaltende Alkyl-, Cycloalkyl-, Alkenyl-, Aryl- oder Aralkylgruppe mit bis zu 18, vorzugsweise 1 bis 12, C-Atomen darstellen, mindestens ein Rest aber eine Alkyl- oder Alkenylgruppe mit 4 bis 22, vorzugsweise 8 bis 22, C-Atomen ist und $X^{(-)}$ ein Monoanion, vorzugsweise das

Chloridion, darstellt.

3. Verfahren nach Anspruch 1, dadurch gekennzeichnet, daß die quartären Phosphoniumverbindungen die allgemeine Formel

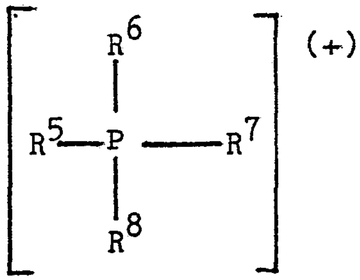

$X^{(-)}$ (II),

haben, in der $R^5$, $R^6$, $R^7$ und $R^8$ gleich oder verschieden sind und Alkyl-, Cycloalkyl-, Alkenyl, Aralkyl- oder Arylgruppen mit jeweils 1 - 12 C-Atomen darstellen, die gegebenenfalls Hydroxyl- oder Äthergruppen enthalten und $X^{(-)}$ ein Monoanion, vorzugsweise das Chloridion, ist.

4. Verfahren nach Anspruch 1, dadurch gekennzeichnet, daß die tertiären Sulfoniumverbindungen die allgemeine Formel

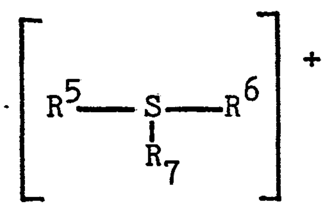

$X^{(-)}$ (III)

haben, in der $R^5$, $R^6$, und $R^7$ gleich oder verschieden sind und Alkyl-, Cycloalkyl-, Alkenyl, Aralkyl- oder Arylgruppen mit 1 - 12 C-Atomen darstellen, die gegebenenfalls Hydroxyl- oder Äthergruppen enthalten und $X^{(-)}$ ein Monoanion, vorzugsweise das Chloridion, ist.

5. Verfahren nach einem oder mehreren der Ansprüche 1 bis 4, dadurch gekennzeichnet, daß der Dehydrohalogenierungskatalysator in einer Menge von 0,01 bis 10, vorzugsweise 0,1 bis 5, Gew.%, bezogen auf Halogenhydrinäther, eingesetzt wird.

6. Verfahren nach einem oder mehreren der Ansprüche 1 bis 7, dadurch gekennzeichnet, daß eine Alkalimenge, vorzugsweise Natronlauge zugegeben wird, deren molares Verhältnis zu der Menge an hydrolysierbarem Halogen oder der Anzahl der Hydroxylgruppen im Ausgangsphenol 0,95 bis 1,5 : 1, vorzugsweise 1,0 bis 1,2 : 1, beträgt.

7. Verfahren nach einem oder mehreren der Ansprüche 1 bis 6, dadurch gekennzeichnet, daß die Temperatur bei der Dehydrohalogenierungsreaktion im Zweistufenverfahren 40 bis 95°C, vorzugsweise 70 bis 90°C, beträgt.

8. Verfahren nach einem oder mehreren der Ansprüche 1 bis 6, dadurch gekennzeichnet, daß die Dehydrohalogenierung beim Einstufenverfahren bei 40 bis 60°C erfolgt.

0031435

Nummer der Anmeldung

Europäisches Patentamt

# EUROPÄISCHER RECHERCHENBERICHT

EP 80 10 7076.4

| EINSCHLÄGIGE DOKUMENTE | | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| D | DE - A - 2 407 092 (INVENTA)<br>* Patentansprüche; Seite 5 Zeilen 13 bis 15; Beispiel 1 *<br>-- | 1,9 | C 07 D 301/28<br>C 08 G 59/06<br>C 09 D 3/58<br>C 09 J 3/16 |
| A | DE - A1 - 2 335 199 (REICHHOLD-ALBERT-CHEMIE)<br>* Beispiel 1 *<br>-- | 1,9 | |
| A | DE - B2 - 2 217 239 (HOECHST)<br>* Spalte 4, Zeilen 12 bis 23, 61, 62; Spalte 8, Zeilen 44, 45 *<br>-- | 1,9 | RECHERCHIERTE SACHGEBIETE (Int. Cl.³)<br>C 07 D 301/02<br>C 07 D 301/27<br>C 07 D 301/28<br>C 08 G 59/00 |
| D,A | DE - A - 2 028 136 (SHELL INTERNATIONALE RESEARCH MAATSCHAPPIJ)<br>* Seite 5, Absatz 2; Seite 6, Zeilen 24, 25; Beispiel 1 *<br>-- | 1,9 | |
| A | US - A - 4 122 067 (ANDERSON)<br>* Spalte 5, Zeilen 22 bis 26 *<br>---- | 10 | |
| | | | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| Berlin | 06-03-1981 | KRAIL |